# EUROPEAN PATENT SPECIFICATION

(11) **EP 1 940 519 B1**
(45) Date of publication and mention of the grant of the patent: **07.03.2012**
(21) Application number: 05795966.0
(22) Date of filing: 09.09.2005
(51) Int. Cl.: A61P 33/06, A61K 31/357, A61K 31/366, A61K 31/00, A61K 31/505, A61K 31/4965

(54) **Pharmaceutical composition comprising artesunate, sulfamethoxypyrazine and pyrimethamine for use in the treatment of malaria within one day**
Pharmazeutische Zusammensetzung enthaltend Artesunat, Sulfamethoxypyrazin und Pyrimethamin zur Verwendung in der Behandlung von Malaria innerhalb eines Tages
Composition pharmaceutique à base d'artésunate, de sulfaméthoxypyrazine et de pyriméthamine pour l'utilisation dans le traitement du palludisme en un jour

(43) Date of publication of application: 09.07.2008
(60) Divisional application: 10192206.0
(73) Proprietor: Dafra Pharma N.V., 2300 Turnhout (BE)
(72) Inventor: PLATTEEUW, Johannes, NL-5283 JH Boxtel (NL); JANSEN, Frans, Herwig, B-2360 Oud-Turnhout (BE)
(74) Representative: van Kooij, Adriaan
(86) International application number: PCT/EP2005/009851
(87) International publication number: WO 2007/028413

(56) References cited:
- ANONYMOUS: "Co-Arinate" DAFRA PHARMA 2004, [Online] 8 March 2005 (2005-03-08), XP002372153 Retrieved from the Internet: URL:http://web.archive.org/web/20050308092 738/http://www.dafra.be/products.php?id=68 > [retrieved on 2006-03-15]
- "Parallel sessions - abstracts of presentations" ACTA TROPICA, ELSEVIER SCIENCE BV., AMSTERDAM, NL, vol. 95, 2005, pages 1-506, XP005041020 ISSN: 0001-706X
- VON SEIDLEIN L ET AL: "Efficacy of artesunate plus pyrimethamine-sulphadoxine for uncomplicated malaria in Gambian children: a double-blind, randomised, controlled trial" LANCET THE, LANCET LIMITED. LONDON, GB, vol. 355, no. 9201, 29 January 2000 (2000-01-29), pages 352-357, XP004820924 ISSN: 0140-6736
- VAN DEN BROEK INGRID ET AL: "Efficacy of two artemisinin combination therapies for uncomplicated falciparum malaria in children under 5 years, Malakal, Upper Nile, Sudan." MALARIA JOURNAL [ELECTRONIC RESOURCE]. 24 FEB 2005, vol. 4, no. 1, 24 February 2005 (2005-02-24), page 14, XP009063433 ISSN: 1475-2875
- HAMOUR S ET AL: "Malaria in the Nuba Mountains of Sudan: baseline genotypic resistance and efficacy of the artesunate plus sulfadoxine-pyrimethamine and artesunate plus amodiaquine combinations" TRANSACTIONS OF THE ROYAL SOCIETY OF TROPICAL MEDICINE AND HYGIENE, ELSEVIER, vol. 99, no. 7, July 2005 (2005-07), pages 548-554, XP004904317 ISSN: 0035-9203
- MOLTA N B ET AL: "EFFICACIES OF CHLOROQUINE PYRIMETHAMINE-SULPHADOXINE AND PYRIMETHAMINE-SULPHALENE AGAINST PLASMODIUM-FALCIPARUM IN NORTHEASTERN NIGERIA" JOURNAL OF TROPICAL MEDICINE AND HYGIENE, vol. 95, no. 4, 1992, pages 253-259, XP009063427 ISSN: 0022-5304
- AMIN A A ET AL: "Brands, costs and registration status of antimalarial drugs in the Kenyan retail sector" MALARIA JOURNAL 26 JUL 2005 UNITED KINGDOM, vol. 4, 26 July 2005 (2005-07-26), XP002372156 ISSN: 1475-2875
- LUXEMBURGER C ET AL: "Single day mefloquine-artesunate combination in the treatment of multi-drug resistant falciparum malaria", TRANSACTIONS OF THE ROYAL SOCIETY OF TROPICAL MEDICINE AND HYGIENE, ELSEVIER, GB, vol. 88, no. 2, 1 March 1994 (1994-03-01), pages 213-217, XP023097868, ISSN: 0035-9203, DOI: DOI:10.1016/0035-9203(94)90303-4 [retrieved on 1994-03-01]
- DAO ET AL: "Fatty food does not alter blood mefloquine concentrations in the treatment of falciparum malaria", TRANSACTIONS OF THE ROYAL SOCIETY OF TROPICAL MEDICINE AND HYGIENE, ELSEVIER, GB, vol. 99, no. 12, 1 December 2005 (2005-12-01), pages 927-931, XP005105637, ISSN: 0035-9203, DOI: DOI:10.1016/J.TRSTMH.2005.04.016
- WIJEYARATNE P M ET AL: "Therapeutic efficacy of antimalarial drugs along the eastern Indo-Nepal border: a cross-border collaborative study", TRANSACTIONS OF THE ROYAL SOCIETY OF TROPICAL MEDICINE AND HYGIENE, ELSEVIER, GB, vol. 99, no. 6, 1 June 2005 (2005-06-01), pages 423-429, XP004852109, ISSN: 0035-9203, DOI: DOI:10.1016/J.TRSTMH.2004.09.011
- [Online] Retrieved from the Internet: <URL:http://www.fightingmalaria.gov/countri es/mops/assessments/uganda_assessment.pdf>
- BIAGINI ET AL: "Current drug development portfolio for antimalarial therapies", CURRENT OPINION IN PHARMACOLOGY, ELSEVIER SCIENCE PUBLISHERS, NL, vol. 5, no. 5, 1 October 2005 (2005-10-01) , pages 473-478, XP005059119, ISSN: 1471-4892, DOI: DOI:10.1016/J.COPH.2005.05.004
- VAN DER MEERSCH H: "L'artémisinine et ses dérivées dans la lutte contre la malaria // Review of the use of artemisinin and its derivatives in the treatment of malaria", JOURNAL DE PHARMACIE DE BELGIQUE, MASSON, PARIS, FR, vol. 60, no. 1, 1 January 2005 (2005-01-01), pages 23-29, XP009145714, ISSN: 0047-2166

## Description

The present invention relates to the use of a pharmaceutical composition suitable for the treatment of malaria within one day.

Drug resistant malaria is a serious clinical and public health problem. Malaria is caused by an infection with the parasite Plasmodium falciparum. This parasite has developed a versatile capacity of evading the effect of many known antimalarial agents either by genetic or by non-genetic methods.

It has been recognized since many years that artemisinin, a sesquiterpene lactone with a peroxide moiety, is a potent antimalarial agent. Artemisinin is usually isolated from Artemisia annua L and many derivatives of artemisinin are known such as artemether, arteether, dihydroartemisinin and artesunate with a similar antimalarial activity.

Artemisinin based monotherapy, a therapy using one active compound, has been extensively used in Asia and Africa in the ongoing battle against Malaria since the discovery of the molecule as an antimalarial agent.

A short treatment course of 3 days is sufficient to cure a patient but reoccurrence in this case is fairly common. Therefore, it was suggested to extend the therapy to 5 or even 7 days. This turned out to give good results and a five day treatment course, if fully completed, leads to cure rates of more than 90 % at follow up for 28 days.

Athough the administration of artemisinin or its derivatives can effectively combats a Plasmodium falciparum infection, one of the drawbacks of this monotherapy is that it takes a minimum of 5 days. A further drawback is that artemisinin or its derivatives are the last line of defense against malaria, making many people concerned about the possibility for development of resistance against these antimalarial agents.

Since a couple of years, in order to avoid the development of drug resistance by the malarial parasite Plasmodium falciparum, it has been recommended by the World Helath Organization (WHO) and other organisations like for example Médecins sans frontières (MSF) to combine an artemisinin monotherapy with one or more drugs, usually also antimalarial agents, having a different mode of action to slow down or to eliminate the development of resistance against these antimalarial agents and further to improve patient compliance by reducing the therapy duration.

Public as well as company based researchers have taken up this challenge and have come up with several possible combinations. Combination therapy exist in co-blisters or Fixed dose formulations of artemisinin or its derivates with a number of other known or unknown antimalarial compounds. Specific examples of these are combinations of artesunate with amodiaquine, of artemether with lumefantrine, of artesunate with mefloquine and suggested are artesunate with chlorproguanil/dapsone and dihydroartemisinin with piperaquine.

Ideally, these combinations should be cheap, easy to administer, safe for children and pregnant women, and the elimination half-lifes of the drugs should match to keep the synergy going for the complete therapeutic interval and the formulation should be stable in tropical conditions.

A promising combination appears to be a combination of artemisinin and two types of antifolate agents such as a dihydropteroate synthase (DHPS) inhibitor and a dihydrofolate reductase (DHFR) inhibitor. A DHPS-inhibitor works by competing with p-aminobenzoic acid for the reactive site on the DHPS-enzyme. A DHFR-inhibitor completely blocks the enzyme in the last step of the folic acid biosynthesis.

Sulfadrugs, like sulfadoxine, sulfamethoxazole, sulfalene and dapsone fall into the category of DHPS inhibitors, whilst the DHFR group is usually represented by trimethoprim, pyrimethamine and or (chlor)proguanil. Although technically speaking antifolate therapy is a combination therapy in itself, the general view is that since the target is the same metabolic pathway.

The application of antifolate therapy to malaria was started by Hoffman la Roche with the introduction of a fixed combination of sulfadoxine and pyrimethamine, better known as Fansidar. Shortly after that Farmitalia, introduced Metakelfin, which is a combination of sulfamethoxypyrazine also known as sulfalene (belonging to the DHPS group) with pyrimethamine (belonging to the DHFR group). However, since Fansidar was the first drug in this class of therapy and the presence of Roche in the marketplace was extensive, Metakelfin never reached the expected sales volume and Fansidar became the predominant antimalarian drug in the market.

Treatment with antifolates has shown reduced efficacy over the last few years, as malaria parasites are known to have developed genetic point mutations which make these drugs less effective. In the discussion about the increasing resistance of the malaria parasite to sulfadrugs, research on Fansidar became instrumental. Unfortunately this has resulted in many generalizations, eventually leading most people to believe that all sulfadrugs are equal.

The above combinations of artemisinin or its derivatives with the two different types of foliate inhibitors and specifically artesunate with sulfadoxine and pyrimethamine provide an effective treatment of malaria within three days. For this, these compounds are usually administered in the form of a tablet comprising artemisinin or its derivatives such as artesunate during three days and the foliate inhibitors sulfadoxine and pyrimethamine are given as a single dose during day 1 of the treatment.

Luxemburger et al. discloses the therapeutic efficacy and toxicity of a combination of low dose mefloquine (15 mg/kg) plus artesunate 10 mg/kg (MA) in one day in comparison with a commonly used regimen of high dose mefloquine (25 mg/kg) (MQ) in 552 patients with uncomplicated *falciparum* malaria in an area of multi-drug resistance on the Thai-Burmese border. MA provided a faster clinical and parasitological responses and prevented early treatment failure. Overall failure rates by day 28 were 19% in the MA group and 24% in with MQ group. There were no evident adverse effect reported associated with artesunate. It was concluded that a single day's treatment with artesunate augments the antimalarial efficacy of mefloquine.

Dao *et al.* compared blood mefloquine concentrations after the administration of artesunate (8 mg/kg) and mefloquine (15 mg/kg) over 12h with either a low-fat (approximately 3g of fat) or high-fat (approximately 30 g of fat) meal for the treatment of *Plasmodium falciparum* malaria in 12 Vietnamese patients. It was reported that a fatty meal does not appear to increase the bioavailability of mefloquine in malaria patients and should not affect the response of malaria infections to treatment.

Wijeyaratne et al. describes a cross-border study to determine the therapeutic efficacy of antimalarial drugs used by the National Programmes for *falciparum* malaria along the eastern Indo-Nepal border where there is unregulated population movement across the border using a WHO 28 day treatment protocol. The efficacy of chloroquine in India and sulfadoxine-pyrimethamine in Nepal was tested in subjects with laboratory-confirmed Plasmodium falciparum malaria. Of the 89 subjects who completed the study in India, there were 46 treatment failures. The authors concluded that, based on WHO guidelines, both countries need to review their drug policy urgently and make appropriate changes, taking into account aspects of cross-border collaboration in the control of drug-resistant malaria.

A report published in 2005 (http://www.fightingmalaria.gov/countires/mops/assessments/u ganda_assessment.pdf) suggests, amongst others, the treatment of malaria using artesunate tablets and sulfamethoxy-pyrimethamine tablets administered over a period of three consecutive days.

Biagini et al. provides an overview of the 2005 drug development portfolio for antimalarial therapies.

However, there is still a need to further reduce the treatment time because of at least four reasons.

First, in order to avoid the occurrence of resistance to the antimalarial agents used, the treatment time should be as short as possible. This because the faster the Plasmodium falciparum parasites are cleared from the system, the less time remains for the parasite to develop drug resistance.

Second, a shorter duration of the treatment would make it more easier to completely finish the treatment. This further decreases the risk of developing resistant Plasmodium falciparum parasites.

Third, a shorter treatment protocol requiring less treatment days can be easier monitored by trained physicians and nurses and in addition allows for the treatment of more people in a local clinic in a shorter period of time.

Fourth, the shorter treatment protocol is more convenient for the patient not in the least because the period of suffering from the disease is reduced.

The above objects are met by the pharmaceutical composition used according to the present invention providing a treatment of malaria in one day.

A pharmaceutical composition is described comprising an antimalarial agent selected from the group consisting of artemether, arteether, artemisinin, dihydroartemisinin and artesunate; sulfamethoxypyrazine (SM); and a dihydrofolate reductase inhibitor pyrimethamine and further comprising one or more pharmaceutical acceptable excipients, carriers and/or diluents.

Sulfamethoxypyrazine is a well-characterized sulfonamide drug belonging to the class of dihydropteroate synthase (DHPS) inhibitors and is chemically very close to sulfadoxine but biologically quite distinct with for example a shorter half-life and less plasma protein binding.

The efficacy of the sulfamethoxypyrazine-dihydrofolate reductase inhibitor combination, and specifically pyrimethamine, in treating Plasmodium falciparum malaria has been demonstrated in the past, although it has not been used widely as an antimalarial for over 10 years. The main problem is that the combination does not reduce the number of gametocytes. This leads to higher transmission rates and also within 28 days 10 to 20 % of the people suffer from a re-infection or recrudescence.

However, in combination with artemisinin, it not only increases the efficacy of the treatment, but, surprisingly, reduces the necessary treatment time to one day.

According to the present invention the antimalarial agent is artesunate. Artesunate is preferred because of his stability and price.

According to the invention, the dihydrofolate reductase inhibitor in pharmaceutical composition is pyrimethamine being the most active dihydrofolate reductase inhibitor.

In the pharmaceutical composition the preferred ratio between the antimalarial agent and the sulfamethoxypyrazine is (50 to 150):(200 to 300), more preferably about 100:250, such as specifically 100:250.

Also preferred is a ratio between the sulfamethoxypyrazine and the dihydrofolate reductase inhibitor of (200 to 300):(10 to 15). In this case the more preferred ratio is about 250:12.5, such as specifically 250:12.5.

Regarding the ratio between the antimalarial agent and the dihydrofolate reductase inhibitor in the pharmaceutical composition the preferred ratio is (50 to 150):(10 to 15), more preferably about 100:12.5, such as specifically 100:12.5.

The pharmaceutical composition can be administered using the common routes of administration such as oral, rectal, parenteral or intramuscular administration routes, however the preferred route of administration is the oral route.

Also, the pharmaceutical composition can be formulated into any known doses form know in the pharmaceutical field such as formulated as a tablet, an injectable solution, a capsule, a granule, a suppository, a powder, a particulate, a micro particulate or a syrup, however, the preferred formulation is a tablet.

Although the specific amounts of active ingredients in the pharmaceutical composition in order to obtain an effective treatment of malaria depend on, amongst others, the condition of the patient, the age of the patient, the disease state, etc, which conditions are in general taken into account by a trained physician. The amounts of active ingredients usually are about 50 mg, 100 mg and 200 mg artesunate, about 125 mg, 250 mg and 500 mg sulfamethoxypyrazine and about 6.1 ng, 12.5 mg and 25 mg pyrimethamine for children, youngsters and adults, respectively.

A pharmaceutical composition kit is described comprising two or three doses forms, wherein the pharmaceutical composition kit comprises the antimalarial agent artesunate; sulfamethoxypyrazine; and a dihydrofolate reductase inhibitor pyrimethamine combination in the two or three doses forms.

This pharmaceutical composition kit provides to the patients suffering from malaria, the active ingredients in two or three doses forms, such as preferably tablets. In case of two doses forms, one doses form can for example comprise the antimalarial agent and sulfamethoxypyrazine and the other the dihydrofolate reductase inhibitor. An other example is wherein one doses form comprises the antimalarial agent and the other doses form comprises the sulfamethoxypyrazine and the dihydrofolate reductase inhibitor. Also a pharmaceutical composition kit can be envisaged comprising two dosage forms whereby one doses form comprises the antimalarial agent and the dihydrofolate reductase inhibitor and the other doses form comprises the sulfamethoxypyrazine. Of course, in case the pharmaceutical composition kit comprises three dosage forms, the active constituents are the single active ingredient of the individual doses form, thus one doses form comprising an antimalarial artesunate, one doses form comprising sulfamethoxypyrazine and one doses form comprising the dihydrofolate reductase inhibitor pyrimethamine.

The pharmaceutical composition kit can comprise a tablet comprising artesunate and a tablet comprising the combination sulfamethoxypyrazine and pyrimethamine.

Depending on the treatment protocol, the above composition of the kit, i.e., two or three dosage forms comprising the active ingredients, can be repeated in the composition kit such as for example a pharmaceutical composition kit comprising three times a tablet comprising artesunate and three times a tablet comprising sulfamethoxypyrazine and pyrimethamine, thereby providing the complete malarial treatment in one pharmaceutical composition kit.

The pharmaceutical composition kit can be further provided with instructions for use, such as for example instructions for use in the form of pictograms depicting the necessary treatment protocol or in written form.

The pharmaceutical composition as described above provides a treatment of malaria within one day through its active constituents. The pharmaceutical composition, or its active constituents, also provides the reoccurrence of the disease during at least a period of 28 days. In addition, also a reinfection with Plasmodium falciparum is prevented during this period providing in addition a prophylaxis for malaria.

Therefore, the present invention relates to a combination of the antimalarial agent, artesunate; sulfamethoxypyrazine and the dihydrofolate reductase inhibitor pyrimethamine or a pharmaceutical composition as described above for use in the prophylaxis and/or treatment of malaria within one day.

According to the present invention, the antimalaria agent is artesunate and the dihydrofolate reductase inhibitor is pyrimethamine.

As stated above, the pharmaceutical composition provides a treatment of malaria within one day through its active constituents, the reoccurrence of the disease during at least a period of 28 days and prevents a reinfection with Plasmodium falciparum during at least this period.

Therefore, the present invention relates to the use of a combination of the antimalarial agent artesunate; sulfamethoxypyrazine and the dihydrofolate reductase inhibitor pyrimethamine or a pharmaceutical composition as described above for the preparation of a medicament for the prophylaxis and/or treatment of malaria within one day.

With respect to the above use, the antimalarial agent is artesunate and the dihydrofolate reductase inhibitor is pyrimethamine.

For an effective treatment using the pharmaceutical composition the use comprises administering a therapeutic effective dose of the combination or the pharmaceutical composition in three intervals in one day. Such therapeutic effective dose is preferably obtained by orally administering three times 100 mg artesunate, 250 mg sulfamethoxypyrazine and 12.5 mg pyrimethamine whereby the administrations are 12 hours apart, thus at the beginning of the treatment, approximately 12 hours later and 24 hours later.

For example, starting in the morning, the treatment protocol can comprise administering about 50 mg, 100 mg and 200 mg artesunate, about 125 mg, 250 mg and 500 mg sulfamethoxypyrazine and about 6.1 mg, 12.5 mg and 25 mg pyrimethamine for children, youngsters and adults, respectively, administering about 50 mg, 100 mg and 200 mg artesunate, about 125 mg, 250 mg and 500 mg sulfamethoxypyrazine and about 6.1 mg, 12.5 mg and 25 mg pyrimethamine for children, youngsters and adults, respectively when the patient goes to sleep, and administering about 50 mg, 100 mg and 200 mg artesunate, about 125 mg, 250 mg and 500 mg sulfamethoxypyrazine and about 6.1 mg, 12.5 mg and 25 mg pyrimethamine for children, youngsters and adults, respectively, when the patient again wakes up, thereby completely curing malaria.

Thus, described is a method for the prophylaxis and/or treatment of malaria comprising administering to an individual a therapeutic effective dose of a combination of an antimalarial agent; sulfamethoxypyrazine and a dihydrofolate reductase inhibitor or a pharmaceutical composition as described above, wherein the antimalarial agent is artesunate and the dihydrofolate reductase inhibitor is pyrimethamine and the therapeutic effective dose is administered in three intervals in one day, thereby providing an effective treatment of an infection with Plasmodium falciparum.

The present invention will be further exemplified in the following examples illustratively demonstrating the efficacy of the invention.

### EXAMPLES

### Example 1: preparation of tablets comprising artesunate-sulfalene-pyrimethamine

**Table 1, dose composition**

| Name | Formula | Function |
|---|---|---|
| Sulfamethoxypyrazine | 250 mg | Active ingredient |
| Pyrimethamine | 12.5 mg | Active ingredient |
| Artesunate | 100 mg | Active ingredient |
| Maltodextrine | 30 mg | Binder, disintegrant |
| Microcrystalline cellulose | 168.1 mg | Filler |
| Colloidal silica anhydrous | 3.4 mg | Flow agent |
| Magnesium stearate | 6 mg | Lubricant |
| Total per tablet | 600 mg | |

### Manufacturing

Sulfamethoxypyrazine, pyrimethamine and maltodextrine were mixed in a granulator. Granulation was started by addition of 20 %(w/w) of water. After granulation and drying, the granulate was passed through a stainless steel sieve. Artesunate, Microcrystalline cellulose and Colloidal silica anhydrous were added and a blend was prepared in a mixer. Subsequently, Magnesium stearate was added and a final blend prepared. The resulting pre-compression mixture was used for tableting.

### Example 2: clinical trial: comparison between artesunate-sulfamethoxypyrazine-pyrimethamine and artemether-lumefantrine

In this example, the efficacy and safety of the combination artesunate-sulfamethoxypyrazine-pyrimethamine was tested against artemether-lumefantrine for the treatment of Plasmodium falciparum malaria.

### Methods

The study was carried out in a peri-urban area of the capital of an African nation, where malaria is mesoendemic and highly seasonal. Subjects aged ≥ 6 months with P. falciparum malaria were randomly assigned to treatment with artesunate-sulfamethoxypyrazine-pyrimethamine or artemether-lumefantrine. Treatment efficacy was assessed using the 28-day WHO 2003 protocol.

Treatment safety was assessed clinically as well as by measuring hematological indices and serum creatinine and hepatic transaminase concentrations. The sample size was computed assuming equal efficacy of the two treatments, and 606 patients (303 in each treatment arm) were enrolled over two consecutive malaria transmission seasons.

### Results

Baseline characteristics were similar between the two treatment groups. No cases of early treatment failure were observed. The cure rate after 28 days was higher for artesunate-sulfamethoxypyrazine-pyrimethamine (98.7%) than artemether-lumefantrine (89.6%), p < 0.0001. However, after correction for cases of re-infection, the 28-day cure rate was 100% for artesunate-sulfamethoxypyrazine-pyrimethamine, compared to 99.0% for artemether-lumefantrine (p=0.3).

Parasite and fever clearance were similar in the two treatments except that after 1 day, 90.8% of those in the artesunate-sulfamethoxypyrazine-pyrimethamine group were without fever versus 77.6% in the artemether-lumefantrine group (p < 0.001). Gametocyte carriage rates were similar in both treatment groups.

Anemia rate decreased significantly in artesunate-sulfamethoxypyrazine-pyrimethamine group after treatment as compared to before treatment (12.7 vs 6.5; p = 0.0003). No serious adverse events or significant laboratory abnormalities occurred.

In the artesunate-sulfamethoxypyrazine-pyrimethamine group, 2/82 (2.4%) and 1/87 (1.2%) as compared to 0/72 and 1/76 (1.3) in artemether-lumefantrine group had mild and transient elevation of transaminases and creatinine, respectively, on day 14 (p > 0.45).

The abnormality of hepatic transaminase and serum creatinine for these subjects resolved spontaneously within 2 weeks, confirming the safety of the pharmaceutical composition according to the present invention.

### Discussion

Administered as a single dose,
artesunate-sulfamethoxypyrazine-pyrimethamine for three-days is at least as well-tolerated and effective as artemether-lumefantrine for the treatment of Plasmodium falciparum malaria.

Artesunate-sulfamethoxypyrazine-pyrimethamine cleared fever faster than artemether-lumefantrine on day 1. Artesunate-sulfamethoxypyrazine-pyrimethamine showed an additional benefit in the prevention of new infections during the 28-day period following treatment and reduced the anemia rates in comparison to artemether-lumefantrine.

### Example 3: Clinical trial: 24 hours treatment protocol versus a 72 hours treatment protocol using artesunate-sulfamethoxypyrazine-pyrimethamine

The efficacy of pharmaceutical compositions containing artesunate, sulfamethoxypyrazine and pyrimethamine was tested in two different treatment regimes.

Subjects aged ≥ 6 months with Plasmodium falciparum malaria were randomly assigned to two different treatment regimes with artesunate-sulfamethoxypyrazine-pyrimethamine. Either 3 doses over 3 days or 3 doses within 24 hours were administered. Treatment efficacy was assessed using the 28-day WHO 2003 protocol. Treatment safety was assessed clinically. The sample size was computed assuming equal efficacy of the two treatments leading to 200 patients (100 in each study arm).

### Results

**Table 2: Patient Baseline characteristics**

| | **24 hours protocol** | **72 hours protocol** |
|---|---|---|
| **number** | 100 | 100 |
| **male/female** | 59/41 | 49/51 |
| **age (years)** | 8.6 (1-43) | 9.6 (1-58) |
| **weight (kg)** | 27.6 (8-79) | 26.8 (6.5-78) |
| **Temperature (°C)** | 39.0 (37.5-41.0) | 38.7 (37.5-41.0) |
| **Parasitaemia** | 17.715 (2000-102640) | 22934 (1200-154000) |

The baseline characteristics of both groups were similar. Only people that could be followed up for 28 days were included.

**Table 3: temperature (°C) after treatment**

| | **24 hours protocol** | **72 hours protocol** |
|---|---|---|
| **day 1** | 37.4 (36.7 - 39.1) | 37.3 (34.4 - 38.5) |
| **day 2** | 37.2 (36.6 - 38.2) | 37.1 (34.2 - 38.0) |
| **day 3** | 37.0 (36.5 - 37.9) | 37.1 (36.5 - 37.7) |
| **day 7** | 37.1 (36.6 - 37.8) | 37.1 (36.5 - 39.0) |
| **day 14** | 37.0 (36.6 - 37.6) | 37.1 (36.5 - 38.0) |
| **day 21** | 37.1 (36.6 - 39.5) | 37.1 (36.6 - 38.7) |
| **day 28** | 37.0 (34.6 - 37.5) | 37.0 (36.5 - 37.6) |

**Table 4: Parasitaemia**

| | **24 hours protocol** | **72 hours protocol** |
|---|---|---|
| **day 1** | 0 | 11 (0-860)* |
| **day 2** | 0 | 0 |
| **day 3** | 0 | 0 |
| **day 7** | 0 | 0 |
| **day 14** | 0 | 0 |
| **day 21** | 0 | 0 |
| **day 28** | 0 | 0 |

| | | |
|---|---|---|
| *only 1 patient was found positive for parasites | | |

No serious adverse events or significant laboratory abnormalities occurred.

### Discussion

The data obtained show that the pharmaceutical composition administered within 24 hours, is as least as efficacious and safe as the pharmaceutical composition administered over 3 days.

## Claims

1. Pharmaceutical composition comprising artesunate, sulfamethoxypyrazine and pyrimethamine and further comprising one or more pharmaceutical acceptable excipients, carriers and/or diluents for use in the prophylaxis and/or treatment of malaria, wherein the prophylaxis and/or treatment comprises administering a therapeutically effective dose of the pharmaceutical composition in three intervals, preferably 12 hours apart, within one day.

2. Pharmaceutical composition for use in the treatment of malaria according to claim 1, wherein the ratio between artesunate and sulfamethoxypyrazine is (50 to 150):(200 to 300), preferably 100:250.

3. Pharmaceutical composition for use in the treatment of malaria according to claim 1 or claim 2, wherein the ratio between sulfamethoxypyrazine and pyrimethamine is (200 to 300):(10 to 15), preferably 250:12.5

4. Pharmaceutical composition for use in the treatment of malaria according to any of the claims 1 to 3, wherein the ration between artesunate and pyrimethamine is (50 to 150):(10 to 15), preferably 100:12.5.

5. Pharmaceutical composition for use in the treatment of malaria according to any of the claims 1 to 4 formulated for oral, rectal, parenteral or intramuscular administration.

6. Pharmaceutical composition for use in the treatment of malaria according to claim 5, wherein the composition is formulated as a tablet, an injectable solution, a capsule, a granule, a suppository, a powder, a particulate, a micro particulate or a syrup, preferably a tablet.

7. Pharmaceutical composition for use in the treatment of malaria according to any of the claims 1 to 6, wherein the therapeutically effective dose is obtained by three times orally administering 50 mg artesunate, 125 mg sulfamethoxypyrazine and 6.1 mg pyrimethamine for children; 100 mg artesunate, 250 mg sulfamethoxypyrazine and 12.5 mg pyrimethamine for youngsters; and 200 mg artesunate, 500 mg sulfamethoxypyrazine and 25 mg pyrimethamine for adults.

## Patentansprüche

1. Pharmazeutische Zusammensetzung, umfassend Artesunat, Sulfamethoxypyrazin sowie Pyrimethamin sowie weiter umfassend eines oder mehrere pharmazeutisch unbedenkliche Hilfs-, Träger- und/oder Verdünnungsmittel, für den Einsatz in der Prophylaxe und/oder Behandlung von Malaria, wobei die Prophylaxe und/oder Behandlung die Verabreichung einer therapeutisch wirksamen Dosis der pharmazeutischen Zusammensetzung in drei Intervallen innerhalb eines Tages, vorzugsweise im Abstand von 12 Stunden, umfasst.

2. Pharmazeutische Zusammensetzung für den Einsatz bei der Behandlung von Malaria nach Anspruch 1, wobei das Verhältnis von Artesunat zu Sulfamethoxypyrazin (50 bis 150):(200 bis 300), vorzugsweise 100:250, beträgt.

3. Pharmazeutische Zusammensetzung für den Einsatz bei der Behandlung von Malaria nach Anspruch 1 oder 2, wobei das Verhältnis von Sulfamethoxypyrazin zu Pyrimethamin (200 bis 300):(10 bis 15), vorzugsweise 250:12,5, beträgt.

4. Pharmazeutische Zusammensetzung für den Einsatz bei der Behandlung von Malaria nach einem der Ansprüche 1 bis 3, wobei das Verhältnis von Artesunat zu Pyrimethamin (50 bis 150):(10 bis 15), vorzugsweise 100:12,5, beträgt.

5. Pharmazeutische Zusammensetzung für den Einsatz bei der Behandlung von Malaria nach einem der Ansprüche 1 bis 4, formuliert für die orale, rektale, parenterale oder intramuskuläre Verabreichung.

6. Pharmazeutische Zusammensetzung für den Einsatz bei der Behandlung von Malaria nach Anspruch 5, wobei die Zusammensetzung als Tablette, injizierbare Lösung, Kapsel, Granulat, Suppositorium, Pulver, Partikel, Mikropartikel oder Sirup, vorzugsweise als Tablette, formuliert ist.

7. Pharmazeutische Zusammensetzung für den Einsatz bei der Behandlung von Malaria nach einem der Ansprüche 1 bis 6, wobei die therapeutisch wirksame Dosis erhalten wird durch dreimalige orale Verabreichung von 50 mg Artesunat, 125 mg Sulfamethoxypyrazin und 6,1 mg Pyrimethamin bei Kindern; 100 mg Artesunat, 250 mg Sulfamethoxypyrazin und 12,5 mg Pyrimethamin bei Jugendlichen sowie 200 mg Artesunat, 500 mg Sulfamethoxypyrazin und 25 mg Pyrimethamin bei Erwachsenen.

## Revendications

1. Composition pharmaceutique comprenant de l'artésunate, de la sulfaméthoxypyrazine et de la pyriméthamine et comprenant en outre un ou plusieurs excipients, supports et/ou diluants pharmaceutiquement acceptables pour une utilisation dans la prophylaxie et/ou le traitement du paludisme, où la prophylaxie et/ou le traitement comprend l'administration d'une dose thérapeutiquement efficace de la composition pharmaceutique en trois fois, de préférence espacées de 12 heures, au sein d'une journée.

2. Composition pharmaceutique pour une utilisation dans le traitement du paludisme selon la revendication 1, dans laquelle le rapport entre l'artésunate et la sulfaméthoxypyrazine est de (50 à 150) / (200 à 300), de préférence 100/250.

3. Composition pharmaceutique pour une utilisation dans le traitement du paludisme selon la revendication 1 ou la revendication 2, dans laquelle le rapport entre la sulfaméthoxypyrazine et la pyriméthamine est de (200 à 300) / (10 à 15), de préférence 250/12,5.

4. Composition pharmaceutique pour une utilisation dans le traitement du paludisme selon l'une quelconque des revendications 1 à 3, dans laquelle le rapport entre l'artésunate et la pyriméthamine est de (50 à 150) / (10 à 15), de préférence 100/12,5.

5. Composition pharmaceutique pour une utilisation dans le traitement du paludisme selon l'une quelconque des revendications 1 à 4, formulée pour une administration orale, rectale, parentérale ou intramusculaire.

6. Composition pharmaceutique pour une utilisation dans le traitement du paludisme selon la revendication 5, où la composition est formulée sous la forme d'un comprimé, d'une solution injectable, d'une capsule, de granulés, d'un suppositoire, d'une poudre, de particules, de microparticules ou d'un sirop, de préférence un comprimé.

7. Composition pharmaceutique pour une utilisation dans le traitement du paludisme selon l'une quelconque des revendications 1 à 6, où la dose thérapeutiquement efficace est obtenue en administrant trois fois par voie orale 50 mg d'artésunate, 125 mg de sulfaméthoxypyrazine et 6,1 mg de pyriméthamine pour les enfants ; 100 mg d'artésunate, 250 mg de sulfaméthoxypyrazine et 12,5 mg de pyriméthamine pour les jeunes ; et 200 mg d'artésunate, 500 mg de sulfaméthoxypyrazine et 25 mg de pyriméthamine pour les adultes.
